# EUROPÄISCHE PATENTSCHRIFT

(11) **EP 3 849 345 B1**
(45) Veröffentlichungstag und Bekanntmachung des Hinweises auf die Patenterteilung: **22.05.2024**
(21) Anmeldenummer: 19769737.8
(22) Anmeldetag: 10.09.2019
(51) Int. Cl.: A23L 33/115, A23D 9/007, C08K 3/36, C10L 5/12, A23L 29/262, A23L 29/294, C10L 7/02, C11C 5/00

(54) **BRENN- UND ESSBARE ZUSAMMENSETZUNG AUS MITTELKETTIGEN TRIGLYCERIDEN UND KIESELSÄURE**
COMBUSTIBLE AND EDIBLE COMPOSITION OF MEDIUM-CHAIN TRIGLYCERIDES AND SILICIC ACID
COMPOSITION COMBUSTIBLE ET COMESTIBLE COMPOSÉE DE TRIGLYCÉRIDES À CHAÎNE MOYENNE ET D'ACIDE SILICIQUE

(30) Priorität: 14.09.2018 DE 102018122533
(43) Veröffentlichungstag der Anmeldung: 21.07.2021
(73) Patentinhaber: Institut Dr. Rilling Healthcare GmbH, 72124 Pliezhausen (DE)
(72) Erfinder: KLAUCK, Wolfgang, 40670 Meerbusch (DE)
(74) Vertreter: Davepon, Björn
(86) Internationale Anmeldenummer: PCT/EP2019/074061
(87) Internationale Veröffentlichungsnummer: WO 2020/053189

(56) Entgegenhaltungen:
- WO-A1-03/063877
- DE-A1-102007 055 341
- US-A1- 2003 054 043
- US-A1- 2003 130 346
- US-A1- 2005 233 044
- Annon: "MCT Oil and MCT Powder", , 17. August 2017 (2017-08-17), Seiten 249-63635, XP055635065, Gefunden im Internet: URL:https://nutridyn.com/pdfs/MET2373-MCT- Oil-Powder-Formula-Focus.pdf [gefunden am 2019-10-23]
- DATABASE GNPD [Online] MINTEL; 14. März 2007 (2007-03-14), anonymous: "Diet Fuel Dietary Supplement", XP055635044, gefunden im www.gnpd.com Database accession no. 673280
- BACH A C ET AL: "MEDIUM-CHAIN TRIGLYCERIDES: AN UPDATE", THE AMERICAN JOURNAL OF CLINICAL NUTRITION, AMERICAN SOCIETY FOR NUTRITION, US, Bd. 36, 1. November 1982 (1982-11-01), Seiten 950-962, XP000856308, ISSN: 0002-9165

## Beschreibung

Die vorliegende Erfindung betrifft eine brenn- und essbare Zusammensetzung enthaltend mittelkettigen Triacylglycerole und Kieselsäure, wobei die Zusammensetzung in einer Ausführungsform ein fester Formkörper ist. Des Weiteren betrifft die Erfindung die Verwendung einer Zusammensetzung oder eines festen Formkörpers als Ausrüstungsgegenstand für Outdooraktivitäten, Camping und/oder sportliche Aktivitäten, und/oder Nahrungsmittel für eine sportliche- oder Outdooraktivität.

Unter mittelkettigen Triacylglycerolen oder Trigliceriden versteht man gemeinhin Triacylglycerole die ungesättigte Fettsäuren mit einer Länge von 6 bis 12 Kohlenstoffatomen enthalten. Diese Triacylglycerole kommen vor allem in Kokosfett, Palmkernöl und Butter vor. Reine mittelkettige Triacylglycerole von Fettsäuren werden durch Hydrolyse von Kokosfett oder Palmkernöl, Fraktionierung der mittelkettigen Fettsäuren und anschließender Veresterung mit Glycerin gewonnen. Reine mittelkettige Triacylglycerole sind unter den Schlagworten Neutralfett, MCT-Öle oder MCT-Fette (medium-chain trigycerides) bekannt und werden in kosmetischen Produkten und als Nahrungsmittel verwendet. Diese Öle sollen bei der Gewichtsreduktion hilfreich sein, die geistige und körperliche Leistungsfähigkeit verbessern und vor verschiedenen Erkrankungen schützen.

Aufgrund ihrer Struktur ist haben MCT-Öle anderer physiologische Eigenschaften als herkömmliche, langkettige Fettsäuren und können einfacher vom Körper aufgenommen werden. MCT-Öle müssen nicht im Darm von Gallensäuren emulgiert werden, damit sie besser verstoffwechselt werden können. Sie bedürfen auch keiner Spaltung durch die Pankreaslipase, einem Enzym der Bauchspeicheldrüse. MCT-Öle werden ohne Umgehung über das Lymphsystem direkt im Blut zur Leber transportiert, wo sie im Vergleich zu herkömmlichen Fetten bevorzugt oxidiert und vermehrt Ketonkörper gebildet werden, welche insbesondere vom Herzen und vom Gehirn als Energiequellen genutzt werden. Aus diesen Gründen liefern MCT-Öle schnell Energie und es ist bekannt, dass sie, beispielsweise in Getränken, für Menschen bei körperlicher Belastung gut verfügbar und nutzbar sind. Außerdem werden MCT-Öle in der klinischen Ernährung zur diätischen Behandlung von verschiedenen Erkrankungen verwendet, wie beispielsweise dem Malabsorptionssyndrom, bei Lymphangiektasien, Morbus Whipple, Chylothorax, exokriner Pankreasinsuffizienz oder im Rahmen einer ketogenen Diät. Die ketogene Diät ist eine kohlenhydratlimitierte, protein- und energiebilanzierte und deshalb fettreiche Form der diätetischen Ernährung, bei welcher der Körper seinen Energiebedarf nur noch aus Fett und daraus im Körper aufgebautem Glukoseersatz, den namensgebenden Ketonkörpern bezieht. Eine ketogene Diät wird als Therapieverfahren vor allem bei Kindern mit pharmakoresistenter Epilepsie, Glukosetransporterstörung wie z.B. GLUT1-Defizit-Syndrom und Pyrovatdehydrogensasemangel eingesetzt. Neuere Forschungsergebnisse lassen auch eine Therapie bei Alzheimererkrankung möglich erscheinen. Des Weiteren werden MCT-Öle auch zur Gewichtsreduktion verwendet.

Herkömmlich werden MCT-Öle angeboten, die üblicherweise als Hauptbestandteile ca. 50-65 % Caprylsäure (C 8) und ca. 30-45 % Caprinsäure (C 10) enthalten. Capronsäure (C 6), Laurinsäure (C12) und Myristinsäure (C14) sind in sehr geringen Anteilen in herkömmlichen Ölen vorhanden Diese MCT-Öle sind u.a. unter den Handelsnamen Miglyol 812^{®} der Firma Sasol, Myritol 312^{®} der Firma Cognis und Tegosoft^{®} CT der Firma Evonik bekannt.

MCT-Öle sind bei Raumtemperatur flüssig und nicht brennbar. Üblicherweise haben MCT-Öle einen Flammpunkt im Bereich von 210°C bis 240°C und eine Zündtemperatur im Bereich von 410 °C bis 440 °C.

Kieselsäure wird als Nahrungsergänzungsmittel verwendet und soll unter anderem das Immunsystem, das Bindegewebe und die Blutgefäße stärken, entzündungshemmend und desinfizierend wirken sowie insbesondere die Struktur von Haaren und Nägeln verbessern. Nahrungsmittel oder Nahrungsergänzungsmittel, denen eine positive Wirkung auf den Körper oder einzelne Körperteile zugeschrieben wird, werden von immer mehr Menschen konsumiert. Eine oftmals speziell auf den eigenen Körper abgestimmte Diät und regelmäßige sportliche Betätigung werden als Teile einer gesunden Lebensweise angesehen, die von immer mehr Menschen angestrebt wird. Zur sportlichen Betätigung gehören häufig nicht nur beispielsweise Besuche im Fitnessstudio, Jogging, Schwimmen oder Krafttraining, sondern auch verschiedene Outdooraktivitäten, wie beispielsweise Fahrradfahren, Mountainbiking, Wandern, Trekking, Bergsteigen, Klettern und Skifahren. Outdooraktivitäten nehmen meist mehrere Stunden oder den ganzen Tag in Anspruch. Sie werden auch bei mehrtätigen Touren oder Ausflügen ausgeübt. Derartige Touren oder Ausflüge werden oft mit Zelten oder Camping verbunden. Gemeinsam ist allen sportlichen- und Outdoor Aktivitäten, dass nach einiger Zeit erste Zeichen körperlicher Erschöpfung und Hunger auftreten. Sofern man sich im Freien betätigt und die Außentemperaturen niedrig oder sogar im Frostbereich sind, fühlt man sich trotz der körperlichen Betätigung kalt. Auch bei einem einfachen Campingaufenthalt, der nicht mit einer Outdooraktivität verbunden ist, muss für Nahrung, die evtl. erwärmt oder gekocht werden muss, und je nach Außentemperatur, Wärme gesorgt werden. Darüber hinaus kann auch die Notwendigkeit oder das Bedürfnis nach einer Lichtquelle bestehen.

Während einerseits die Bedürfnisse nach Nahrung, Wärme und/oder Licht befriedigt werden müssen, ist es auf der anderen Seite nicht gewünscht, schwere oder sperrige Ausrüstungsgegenstände bei einer sportlichen- oder Outdooraktivität oder beim Campen mitzunehmen. Dies gilt bei einem Tagesausflug genauso wie bei einer mehrtätigen Tour. Teilweise ist es auch nicht möglich schwere oder sperrige Ausrüstungsgegenstände mitzunehmen, weil die Möglichkeit oder die individuelle Fähigkeit Lasten zu befördern begrenzt sind. So kann beispielsweise in einem Boot oder Kanu nur Ausrüstung mit einem begrenzten Gewicht und mit einem relativ kleinen Volumen befördert werden. Außerdem sollten Ausrüstungsgegenstände für Outdooraktivitäten oder Campen einfach zu bedienen sein und auf die Anforderungen einer Benutzung im Freien ausgelegt sein. Beispielsweise sollten Wärme- oder Lichtquellen bei Wind nicht erlöschen. Außerdem sollten brennbare Materialien bei der Verbrennung möglichst wenig Ruß oder Rauch bilden, damit sie auch in einem kleinen Raum, wie beispielsweise einem Zelt, verwendet werden können.

Nahrungsmittel und auch brennbare Materialien für eine Outdooraktivität sollten möglichst nicht flüssig sein. Wenn Flüssigkeit ausläuft, werden die umliegenden Gegenstände nass und werden im schlimmsten Fall beschädigt. Insbesondere bei niedrigen Außentemperaturen kann es nicht immer möglich sein feuchte Gegenstände zu trocknen. Sofern Flüssigkeiten mitgenommen werden müssen, sollten sie so verpackt sein, dass sie nicht auslaufen. Eine reiß- und bruchfeste Verpackung geht jedoch häufig mit einem erhöhten Gewicht einher.

Es besteht ein Bedarf an Ausrüstungsgegenständen für sportliche- oder Outdooraktivitäten und/oder Campen die den Anforderungen genügen, welche durch die begrenzten Möglichkeit oder die begrenzte individuelle Fähigkeit Lasten zu befördern, an sie gestellt werden. Diese Anforderungen können durch leichte und kleine Ausrüstungsgenstände befriedigt werden. Die Anforderungen können jedoch auch durch einen Ausrüstungsgegenstand erfüllt werden, der unterschiedliche Funktionen hat. Ein mehrfunktionaler Ausrüstungsgegenstand kann anderer Ausrüstungsgegenstände sogar ersetzen und auf diese Weise zur Verringerung der Lastengewichts beitragen. Außerdem besteht ein Bedarf an Nahrungsmitteln oder brennbaren Materialien für eine sportliche- oder Outdooraktivität die nicht flüssig sind. Außerdem besteht ein Bedarf für Nahrungsmittel, die in der therapeutische Ernährung zur diätischen Behandlung von Erkrankungen oder zur Gewichtsreduktion verwendet werden können und für Patienten und/oder das medizinische Personal einfach und angenehm in der Verwendung sind, weil sie leicht zu dosieren sind und einen angenehmen Geschmack haben.

Aufgabe der vorliegenden Erfindung ist es, eine Zusammensetzung zur Verfügung zu stellen, die als Nahrungsmittel und auch als brennbares Material verwendet werden kann. Vorzugsweise soll die Zusammensetzung, nachdem sie angezündet wurde, so verbrennen, dass die Flamme nicht durch Ausblasen mit der Atemluft gelöscht werden kann, also auch bei starkem Wind nicht erlischt. Des Weiteren soll bei der Verbrennung vorzugsweise kaum Ruß gebildet werden, damit die Zusammensetzung auch in geschlossenen Räumen, insbesondere auch in einem Zelt, als Licht- und Wärmequelle verwendet werden kann. Vorzugsweise ist die Zusammensetzung bei Raumtemperatur ein Gel, ein gelartiger Formkörper oder ein Pulver. Darüber hinaus ist es Aufgabe eine Zusammensetzung zur Verfügung zu stellen, die im Gesundheitsbereich, insbesondere in der medizinischen oder klinischen Ernährung zur diätischen Behandlung von Erkrankungen verwendet werden kann und für Patienten und/oder das medizinische Personal einfach und angenehm zu verwenden ist, insbesondere im Vergleich zu herkömmlichen, flüssigen Produkten.

Diese Aufgabe wird erfindungsgemäß gelöst durch eine Zusammensetzung enthaltend oder bestehend aus
- von 70 bis 96 Gew.-% Triacylglycerol von gesättigten Fettsäuren, welche eine Alkylkette von 6 bis 12 Kohlenstoffatomen enthalten,
- von 3,0 bis 30 Gew.-% an Kieselsäure, und
- optional von 0,1 bis 5,0 Gew.-% wenigstens eines Verdickungsmittels enthaltend wenigstens ein Cellulosederivat und/ oder Wasser,
- optional von 1,0 bis 35 Gew.-% wenigstens eines Zusatzstoffes, ausgenommen Verdickungsmittel,

jeweils bezogen auf das Gesamtgewicht der Zusammensetzung, und
das Verhältnis von Triacylglycerol zu Kieselsäure in einem Bereich von 1 : 0,04 bis 1 : 0,20 liegt, wobei die Zusammensetzung dadurch gekennzeichnet ist, dass
die Kieselsäure pyrogene Kieselsäure und/oder gefällte Kieselsäure ist, wobei wenn die Kieselsäure pyrogene Kieselsäure ist oder diese umfasst und in der Zusammensetzung bis zu 4,0 Gew.-% hiervon enthalten sind, die Zusammensetzung 0,1 bis 5,0 Gew.-% des wenigstens einen Verdickungsmittels enthält und
wenn die Kieselsäure gefällte Kieselsäure ist oder diese umfasst und in der Zusammensetzung bis zu 10 Gew.-%, hiervon enthalten sind, die Zusammensetzung 0,1 bis 5,0 Gew.-% des wenigstens einen Verdickungsmittels enthält,
wobei die Zusammensetzung eine dynamische Viskosität nach DIN 53018 von 50.000 bis 400.000 mPa·s, gemessen mit einem Brookfield Viskosimeter, Spindel 5, bei 0,5
Umdrehungen/min bei 21 °C aufweist oder bei Raumtemperatur fest ist und einen Flammpunkt nach EN 22719 von 210°C bis 240°C hat.

Es wurde überraschend gefunden, dass die Zugabe von Kieselsäure zu mittelkettigen Triacylglycerolen dazu führt, dass mittelkettige Triacylglycerole entzündet werden können und nach der Entzündung vollständig verbrennen. Des Weiteren wurden überraschend gefunden, dass die erfindungsgemäße Mischung weitgehend rußfrei verbrennt und die Flamme nicht durch Ausblasen mit der Atemluft gelöscht werden kann, also auch bei starkem Wind nicht erlischt. Da sowohl Kieselsäure als auch Triacylglycerol essbar sind, kombiniert die erfindungsgemäße Zusammensetzung zwei Eigenschaften, nämlich Essbarkeit und Brennbarkeit.

Ein weiterer Vorteil der vorliegenden Erfindung ist, dass die Zusammensetzung auch als Ersatz für einen Gaskocher verwendet werden kann, da sie nicht nur Licht zur Verfügung stellt, sondern auch ausreichend Wärme um Nahrung oder Flüssigkeiten zu erhitzen. Außerdem kann die Zusammensetzung auch als Bratfett verwendet werden. Man kann z.B. eine Pfanne damit einreiben und auf diese Weise vor dem Braten eines Nahrungsmittels einfetten.

Bevorzugt enthält oder besteht die nicht flüssige Zusammensetzung aus
- von 84 bis 96 Gew.-%, bevorzugter 84 bis 92 Gew.-% Triacylglycerol von gesättigten Fettsäuren, welche eine Alkylkette von 6 bis 12 Kohlenstoffatomen enthalten,
- von 4,0 bis 20 Gew.-%, bevorzugter 4,0 bis 15 Gew.-%, an Kieselsäure, und
- optional höchstens 5,0 Gew.-%, bevorzugter 0,2 bis 0,4 Gew.-% des wenigstens eines Verdickungsmittels enthaltend wenigstens ein Cellulosederivat und/ oder Wasser,
- optional von 4,0 bis 10 Gew.-% des wenigstens eines Zusatzstoffes, ausgenommen Verdickungsmittel,

jeweils bezogen auf das Gesamtgewicht der Zusammensetzung, und
das Verhältnis von Triacylglycerol zu Kieselsäure in einem Bereich von 1 : 0,04 bis 1 : 0,20, bevorzugt von 1 : 0,10 bis 1: 0,20 liegt.

Unter einer Kieselsäure werden im Rahmen der Erfindung die oligomeren und polymeren Kondensationsprodukte der Monokieselsäure Si(OH)₄ verstanden. Kieselsäure kann sowohl als uneinheitlich gebaute amorphe Kieselsäure als auch als kristallisierte Kieselsäure vorliegen. Des Weiteren sind nach dieser Erfindung auch sog. Fällungskieselsäuren von dem Begriff der Kieselsäure umfasst. Unter Fällungskieselsäuren versteht man wässrige Lösungen der amorphen Kieselsäure, sog. Kieselsole, und angesäuerte, halbfeste Kieselsole, sog. Kieselgele. Auch getrocknete Kieselgele sind als Kieselsäure im Sinne der Erfindung anzusehen (Hollemann, Wiberg, Lehrbuch der Anorganischen Chemie, 101. Auflage, 1995, S. 919 ff.). Darüber hinaus werden pyrogene Kieselsäuren von dem Begriff der Kieselsäure umfasst. Pyrogene Kieselsäure ist ein amorphes SiO₂-Pulver das häufig durch Flammenhydrolyse von Silanen oder SiCl₄ gewonnen wird.

Erfindungsgemäß enthält die Zusammensetzung pyrogene Kieselsäure und/oder gefällte Kieselsäure.

Erfindungsgemäß enthält die Zusammensetzung pyrogene Kieselsäure, bevorzugt 4,0 bis 30 Gew.-%, bevorzugter 10 bis 20 Gew.-% pyrogene Kieselsäure und/oder gefällte Kieselsäure, bevorzugt 10 bis 15 Gew.-%, bevorzugter 11 bis 13 Gew.-% gefällte Kieselsäure, jeweils bezogen auf das Gesamtgewicht der Zusammensetzung.

Bevorzugt beträgt der Wassergehalt der Kieselsäure nach DIN EN ISO 787-2 höchstens 3,0 Gew.-% Wasser, bevorzugter von 0,1 Gew.-% bis 0,5 Gew.-%, noch bevorzugter 0,1 Gew.-% bis 0,3 Gew.-%, jeweils bezogen auf die Gesamtmenge Kieselsäure in der Zusammensetzung. Bevorzugt weist die Kieselsäure eine BET-Oberfläche nach DIN ISO 9277 von 150 m²/g bis 250 m²/g, insbesondere von 160 m²/g bis 200 m²/g, auf.

Unter Triacylglycerol von gesättigten Fettsäuren wird im Rahmen der Erfindung Triacylgycerol von ungesättigten Fettsäuren mit einer Länge von 6 bis 12 Kohlenstoffatomen verstanden. Bevorzugt ist das Triacylglycerol in der erfindungsgemäßen Zusammensetzung ein reines mittelkettiges Triacylgycerol, insbesondere gewonnen durch Hydrolyse von Kokosfett oder Palmkernöl, Fraktionierung der mittelkettigen Fettsäuren und anschließender Veresterung mit Glycerin. Reine mittelkettige Triacylglycerole werden auch Neutralfett, MCT-Öl oder MCT-Fette (medium-chain trigycerides) genannt.

Bevorzugt beträgt der Wassergehalt des Triacylglycerols höchstens 0,08 Gew.-% Wasser, bevorzugter höchstens 0,07 Gew.-%, noch bevorzugter höchstens 0,05 Gew.-%, jeweils bezogen auf die Gesamtmenge Triacylgycerol in der Zusammensetzung. In einer bevorzugten Ausführungsform enthält das Triacylglycerol höchstens 0,05 Gew.-% Wasser und folgende Fettsäuren
- von 50 bis 63 Gew.-% Caprylsäure (C 8),
- von 34 bis 43 Gew.-% Caprinsäure (C 10),
- von 0,5 bis 2,0 Gew.-% Laurinsäure (C12),
- von 0,5 bis 1,0 Gew.-% Capronsäure (C6), und
- von 0,5 bis 1,0 Gew.-% Myristinnsäure (C14), jeweils bezogen auf die Gesamtmenge an Fettsäuren.

Die Zusammensetzung enthält optional ein Verdickungsmittel. Insbesondere bei Zusammensetzungen, die eine vergleichsweise geringe Konzentration an gefällter oder pyrogener Kieselsäure enthalten, sind durch die Zugabe eines Verdickungsmittels auch brennbare Zusammensetzungen erhältlich, die ohne die Zugabe des Verdickungsmittels nicht brennbar wären. Zusammensetzungen mit nur einer geringen Konzentration an Kieselsäure enthalten erfindungsgemäß:
- bis zu 4,0 Gew.-% pyrogener Kieselsäure und 0,1 bis 5,0 Gew.-% wenigstens eines Verdickungsmittels, oder
- bis zu 10 Gew.-% gefällter Kieselsäure und 0,1 bis 5,0 Gew.-% wenigstens eines Verdickungsmittels, jeweils bezogen auf das Gesamtgewicht der Zusammensetzung.

In einer erfindungsgemäßem Ausführungsform enthält das Verdickungsmittel wenigstens ein Cellulosederivat und/ oder Wasser. In einer anderen bevorzugten Ausführungsform ist das Verdickungsmittel bei Raumtemperatur ein Feststoff, insbesondere ein Pulver. Der Feststoff oder das Pulver können bevorzugt in mindestens einem Lösungsmittel gelöst sein. Dabei ist insbesondere bevorzugt eine 5 %ige Mischung von Ethylcellulose in Toluol und Ethanol in einem Mischungsverhältnis von 80 : 20. Bevorzugt umfasst das Verdickungsmittel weitere Verdickungsmittel, ausgewählt aus der Gruppe bestehend aus Cellulosefasern, Alginaten, Agar-Agar, Johannisbrotkernmehl, Guarkernmehl, Carrageen, Furcellaran, Xanthan, Pektin, Methylcellulose, Ethylcellulose, Hydroxyethylcellulose, Carboxymethylcellulosen, Hydroxypropylmethyl-cellulose, Hydroxypropylcellulose, Ethylhydroxyethylcellulose und mikrokristalliner Cellulose sowie Mischungen von diesen. Cellulosederivate sind chemische Derivate von Cellulose, die beispielsweise durch Methylierung, Ethylierung, Hydroxypropylierung, Acetylierung und Oxidation gewonnen werden. Bevorzugt ist das Verdickungsmittel Ethylcellulose. Ethylcellulose ist typischerweise ein weißes Pulver und löslich in organischen Lösungsmitteln. Bevorzugt enthält die erfindungsgemäße Zusammensetzung Ethylcellulose mit einem Ethoxylierungsgrad von mindestens 60 %, bevorzugter mindestens 45 %.

In einer anderen erfindungsgemäßen Ausführungsform enthält die Zusammensetzung kein Verdickungsmittel. In Zusammensetzungen, die kein Verdickungsmittel enthalten, beträgt das Verhältnis von MCT-Öl zu SiO₂ bevorzugt 1 : 0,15 bis 1 : 0,5. Bevorzugt enthalten Zusammensetzungen ohne Verdickungsmittel 6-10 Gew.-% pyrogene Kieselsäure oder 12-15 Gew.-% gefällte Kieselsäure, jeweils bezogen auf das Gesamtgewicht der Zusammensetzung. Eine bevorzugte Ausführungsform ohne Verdickungsmittel enthält 80 bis 85 Gew.-% MCT-Öl und 10 bis 15 Gew.-% pyrogene Kieselsäure, jeweils bezogen auf das Gesamtgewicht der Zusammensetzung.

Die Zusammensetzung kann optional einen weiteren Zusatzstoff, der kein Verdickungsmittel ist, enthalten. Bevorzugt enthält eine erfindungsgemäße Zusammensetzung von 4,0 bis 6,0 Gew.-% des wenigstens einen Zusatzstoffes, ausgenommen Verdickungsmittel, bezogen auf das Gesamtgewicht der Zusammensetzung. Bevorzugt ist der Zusatzstoff ausgewählt aus der Gruppe bestehend aus
- Nahrungsergänzungsmitteln gemäß den Anlagen 1 und 2 zur Verordnung über Nahrungsergänzungsmittel, vom 24.05.2004, BGBl. I S. 1011, Stand 17.1.2007,
- Lebensmittelzusatzstoffen gemäß Anlage 1 zur Verordnung über die Zulassung von Zusatzstoffen zu Lebensmitteln zu technologischen Zwecken, vom 29.01.1998, BGBl. I S. 230, Stand 05.07.2017, und
- Aromen gemäß der Aromenverordnung vom 2. Mai 2006 (BGBl. I S. 1127), Stand 05.07.2017, sowie Mischungen von diesen.

Nahrungsergänzungsmittel im Sinne der Nahrungsmittelergänzungsverordnung sind Vitamine und Mineralstoffe, einschließlich Spurenelementen. Bevorzugt sind als Nahrungsergänzungsmittel Vitamine und Mineralien, insbesondere ausgewählt aus der Gruppe bestehend aus Vitamin C, Vitamin D3, Vitamin K, Calcium- und Magnesiumsalzen sowie Mischungen hiervon. Ein bevorzugtes Vitamin ist Vitamin C ausgewählt aus der Gruppe bestehend aus L-Ascorbinsäure, Natrium-L-Ascorbat, Calcium-L-Ascorbat, Calium-L-Ascorbat und L-Ascorbyl-6-Palmitat sowie Mischungen hiervon. Ein weiteres bevorzugtes Vitamin ist Vitamin D in Form von Cholecalciferol oder Ergocalciferol. Ein weiteres bevorzugtes Vitamin ist Vitamin K, bevorzugt als Phyllochinon. Des Weiteren sind bevorzugte Nahrungsergänzungsmittel Calcium- und Magnesiumsalze bevorzugt ausgewählt aus der Gruppe bestehend aus Calciumcarbonat, Calciumchlorid, Calciumsalzen der Zitronensäure, Calciumgluconat, Calciumglycerophosphat, Calciumlactat, Calciumsalze der Orthophosphorsäure, Calciumhydroxid, Calciumoxid, Magnesiumacetat, Magnesiumcarbonat, Magnesiumchlorid, Magnesiumsalze der Zitronensäure, Magnesiumgluconat, Magnesiumglycerophosphat, Magnesiumsalze der Orthophosphorsäure, Magnesiumlactat, Magnesiumhydroxid, Magnesiumoxid und Magnesiumsulfat sowie Mischungen hiervon.

Lebensmittelzusatzstoffe sind Verbindungen, die Lebensmitteln zur Erzielung chemischer, physikalischer oder auch physiologischer Effekte zugegeben werden. Sie werden eingesetzt, um Struktur, Geschmack, Geruch, Farbe und chemische und mikrobiologische Haltbarkeit verarbeiteter Lebensmittel, also ihren Gebrauchs- und Nährwert zu regulieren bzw. zu stabilisieren sowie die störungsfreie Produktion der Lebensmittel sicherzustellen. Die Verordnung über die Zulassung von Zusatzstoffen zu Lebensmitteln zu technologischen Zwecken, kurz Zusatzstoff-Zulassungsverordnung (ZZulV), regelt die Zulassung, Kennzeichnung und Höchstmengen von Zusatzstoffen zu Lebensmitteln. Als Lebensmittelzusatzstoff enthält die Zusammensetzung bevorzugt Farbstoffe, Konservierungsstoffe, Süßungsmittel und/oder Antioxidantien.

Bevorzugt enthält die Zusammensetzung Konservierungsstoffe ausgewählt aus der Gruppe bestehend aus Sorbinsäure, Kaliumsorbat, Calciumsorbat, Benzoesäure, Natriumbenzoat, Kaliumbenzoat, Calciumbenzoat, 4-Hydroxybenzoesäure-ethylester, Natrium-4-Hydroxybenzoesäure-ethylester, 4-Hydroxybenzoesäure-methylester, Schwefeldioxid, Natriumsulfit, Natriumhydrogensulfit, Natriumsdisulfit, Kaliumdisulfit, Calciumdisulfit, Calciumhydrogensulfit, Kaliumhydrogensulfit, Äpfelsäure, Fumarsäure und Lysozym sowie Mischungen hiervon.

Bevorzugt enthält die Zusammensetzung Süßungsmittel ausgewählt aus der Gruppe bestehend aus Sorbit, Mannit, Acesulfam K, Aspartam, Cyclamat, Isomalt, Saccharin, Sucralose, Thaumatin, Neohesperidin-Dihydrochalkon, Stevioglycoside, Neotam, Maltit, Lactitol, Xylitol, Erythrit und Advantam sowie Mischungen hiervon.

Bevorzugt enthält die Zusammensetzung Antioxidantien ausgewählt aus der Gruppe bestehend aus Ascorbinsäure, Natriumascorbat, Calciumascorbat, Ascorbylpalmitat, Ascorbylsteatrat, Alpha-Tocopherol, Gamma-Tocopherol, Delta-Tocopherol, Propylgallat, Octylgallat, Dodecylgallat, Isoascorbinsäaure, Natriumisoascorbat, tert-Butylhydrochinon, Citronensäure, Phosphate, Posphorsäure, Bernsteinsäure und Rosmarinextrakt sowie Mischungen hiervon.

Aromen sind Erzeugnisse, die Lebensmitteln zugesetzt werden um ihnen einen besonderen Geruch und/oder Geschmack zu verleihen oder zu verändern. Bevorzugt enthält die Zusammensetzung Aromen ausgewählt aus der Gruppe bestehend aus Aromastoffen, natürlichen Aromastoffen, Aromaextrakten, thermisch gewonnenen Reaktionsaromen, Raucharomen, Aromavorstufen und sonstige Aromen oder Mischungen hiervon. Bevorzugt enthält die Zusammensetzung Aromen mit einer fruchtigen Geschmacksrichtung. Dazu zählen bevorzugt Geschmacksrichtungen nach Zitrusfrüchten ausgewählt aus der Gruppe bestehend aus Orangen, Blutorangen, Grapefruit, Mandarinen, Zitronen und Kumquats. Besonders bevorzugt sind die Geschmacksrichtungen Orangen und Zitrone, insbesondere als Zitronenöl oder Orangenöl. Weitere bevorzugte Geschmacksrichtungen sind Beeren, Heidelbeere, Himbeere, Erdbeere, Apfel, Birne, Trauben und Melonen. Des Weiteren sind Vanille- und Schokoladengeschmack bevorzugte Aromen.

Bevorzugt enthält die erfindungsgemäße Zusammensetzung ausschließlich Bestandteile, die jeweils zugelassen sind
- als Lebensmittel gemäß dem Lebensmittel und Futtermittelgesetzbuch (LFGB) sowie der Verordnung EG 178/2002 (Lebensmittel-Basisverordnung), oder
- als Nahrungsergänzungsmittel gemäß den Anlagen 1 und 2 zur Verordnung über Nahrungsergänzungsmittel, vom 24.05.2004, BGBl. I S. 1011, Stand 17.1.2007, oder
- als Lebensmittelzusatzstoffe gemäß Anlage 1 zur Verordnung über die Zulassung von Zusatzstoffen zu Lebensmitteln zu technologischen Zwecken, vom 29.01.1998, BGBl. I S. 230, Stand 05.07.2017, oder
- als Aromen gemäß der Aromenverordnung vom 2. Mai 2006 (BGBl. I S. 1127), Stand 05.07.2017. Eine bevorzugte Ausführungsform der Zusammensetzung enthält oder besteht aus
- von 85 bis 90 Gew.-% Triacylglycerol von gesättigten Fettsäuren, welche eine Alkylkette von 6 bis 12 Kohlenstoffatomen enthalten,
- von 4,0 bis 15 Gew.-% an Kieselsäure, insbesondere pyrogene Kieselsäure,
- optional von 0,1 bis 5,0 Gew.-% wenigstens eines Verdickungsmittels, insbesondere einer Mischung aus 3 Gew.- % Ethylcellulose mit 0,2 Gew.-% Wasser, und
- optional 0,1 bis 7,0 Gew.-% des wenigstens einen Zusatzstoffes, ausgenommen Verdickungsmittel, jeweils bezogen auf das Gesamtgewicht der Zusammensetzung.

Die erfindungsgemäße Zusammensetzung ist brennbar und hat einen Flammpunkt nach EN 22719 von 210°C bis 240°C. Bevorzugt brennt eine würfelförmige Masse von 50 g bestehend aus der erfindungsgemäßen Zusammensetzung mindestens 10 Minuten. Bevorzugt hat die erfindungsgemäße Zusammensetzung einen Flammpunkt nach EN 22719 von 220 bis 230 °C und brennt insbesondere nach Wegnahme der Zündquelle selbstständig weiter. Bevorzugt verbrennt eine Zusammensetzung vollständig nach Wegnahme der Zündquelle selbstständig, dh. bis zu dem Zeitpunkt an dem kein MCT-Öl mehr vorhanden ist. Der Flammpunkt eines Stoffes ist die niedrigste Temperatur, bei der sich über einem Stoff ein zündfähiges Dampf-Luft-Gemisch bilden kann. Aufgrund ihrer Brennbarkeit kann die Zusammensetzung als Licht- und Wärmequelle eingesetzt werden. Durch den Flammpunkt der erfindungsgemäßen Zusammensetzung in einem Temperaturbereich, der dem einer Feuerzeugflamme entspricht, kann die Zusammensetzung ohne besondere Ausrüstung als Licht- und Wärmequelle eingesetzt werden. Beispielsweise kann sie anstelle eines Gaskochers beim Camping verwendet werden um Flüssigkeiten und Nahrung zu erhitzen. Bevorzugt ist für das Verbrennen der erfindungsgemäßen Zusammensetzung außerhalb von geschlossenen Räumen kein Windschutz für die Flamme notwendig. Des Weiteren kann die Zusammensetzung auch als Fett zum Braten von Nahrungsmitteln verwendet werden, in dem eine Pfanne mit der Zusammensetzung eingerieben wird und dann ein Nahrungsmittel in dieser Pfanne gebraten wird.

Erfindungsgemäß weist die Zusammensetzung eine dynamische Viskosität nach DIN 53018 von 50.000 bis 400.000 mPa·s, insbesondere von 70.000 bis 350.000 mPa·s, auf, gemessen mit einem Brookfield Viskosimeter, Spindel 5, bei 0,5 Umdrehungen pro Minute bei 21 °C oder sie ist bei Raumtemperatur fest. Eine bevorzugte Ausführungsform hat eine Viskosität von höchstens 80.0000 mPa·s. Eine andere bevorzugte Ausführungsform hat eine Viskosität von höchsten 360.0000 mPa·s. Bevorzugt ist die erfindungsgemäße Zusammensetzung bei Raumtemperatur fest. Als feste Zusammensetzungen im Sinne dieser Erfindung werden Zusammensetzungen angesehen, die nach Beendigung des Herstellungsprozesses ihre Form bei Raumtemperatur nicht mehr allein durch Einwirkung der Schwerkraft verändern, wobei zur Prüfung dieser Eigenschaft ein Würfel mit 1 cm Kantenlänge verwendet werden soll. Ein derartiger Würfel aus einer erfindungsgemäßen festen Zusammensetzung fließt also nicht auseinander, fällt nicht in sich zusammen oder bildet keine mit dem Auge sichtbare partielle Ausbuchtungen oder Beulen. Bevorzugt kann eine feste Zusammensetzung auch ein festes Gel sein. Bevorzugt ist die Viskosität von festen Gelen im Sinne dieser Erfindung nicht bei Raumtemperatur messbar vielmehr zerbrechen Formkörper, die von erfindungsgemäßen festen Gelen gebildet werden, bei mechanischer Einwirkung. In einer weiteren bevorzugten Ausführungsform ist die erfindungsgemäße Zusammensetzung ein Pulver.

Eine weitere bevorzugte Ausführungsform der Zusammensetzung betrifft einen festen Formkörper enthaltend die oben beschriebene erfindungsgemäße Zusammensetzung, wobei der Formkörper bevorzugt teilweise oder vollständig mit einer Beschichtung überzogen ist. Bevorzugt enthält oder besteht die Beschichtung aus Alkylcellulose, bevorzugt ist die Alkylcellulose eine Ethylcellulose. Die Beschichtung verhindert, dass sich der Formkörper glitschig, klebrig oder fettig anfühlt, ohne jedoch die Brennbarkeit des Formkörpers zu beeinträchtigen. Des Weiteren stellt die Beschichtung sicher, dass eine Verpackung einfach vom Formkörper abgelöst werden kann und nicht an dem Formkörper klebt, wenn die Verpackung geöffnet wird. Des Weiteren verhindert die Beschichtung, dass Flüssigkeiten, insbesondere Wasser, in den Formkörper eindringen können.

Einige Bestandteile der Zusammensetzung haben mehr als eine Funktion. Beispielsweise können Verdickungsmittel wie Ethylcellulose auch als Beschichtung für den festen Formkörper dienen. Andere Bestandteile können als Nahrungsergänzungsmittel und/oder Lebensmittelzusatzstoff und/oder Aroma angesehen werden.

Die Erfindung betrifft auch die Verwendung einer erfindungsgemäßen Zusammensetzung oder eines erfindungsgemäßen Formkörpers als Ausrüstungsgegenstand für Outdoor-Aktivitäten, Camping und/oder sportlichen Aktivitäten und/oder Nahrungsmittel für eine sportliche- oder Outdooraktivität. Dabei sind sportliche Aktivitäten und/oder Outdoor-Aktivitäten beispielsweise Jogging, Schwimmen, Fahrradfahren, Mountainbiking, Wandern, Trekking, Paddeln, Rudern, Rafting, Skifahren, Tennis, Fußball, Handball, Volleyball, Basketball, Bergsteigen, Klettern und Krafttraining. Eine Verwendung im Gesundheitsbereich betrifft vorzugsweise die therapeutische Ernährung zur diätischen Behandlung von verschiedenen Erkrankungen oder zur Gewichtsreduktion, insbesondere die therapeutische Ernährung bei dem Malabsorptionssyndrom, bei Lymphangiektasien, bei Morbus Whipple, bei Chylothorax, beiexokriner Pankreasinsuffizienz oder im Rahmen einer ketogenen Diät, insbesondere die ketogene Diät als Therapieverfahren vor allem bei Kindern mit pharmakoresistenter Epilepsie, Glukosetransporterstörung, wie z.B. GLUT1-Defizit-Syndrom, und Pyrovatdehydrogensasemangel sowie auch bei Alzheimererkrankungen.

Eine Ausführungsform des Ausrüstungsgegenstandes enthält einen beschichteten Formkörper, eine andere Ausführungsform enthält einen unbeschichteten Formkörper. Bevorzugt ist der Ausrüstungsgegenstand für Outdoor-Aktivitäten in eine Verpackung eingepackt, wobei die Verpackung vorzugsweise aus einem brennbaren Material besteht. Dadurch kann die Verpackung zusammen mit der Zusammensetzung verbrannt werden. In einer anderen bevorzugten Ausführungsform ist die Verpackung des Ausrüstungsgegentandes kompostierbar oder anderweitig biologisch abbaubar.

### Beispiele

Die vorliegende Erfindung wird anhand der nachfolgenden Beispiele weiter erläutert, ohne jedoch darauf beschränkt zu sein.

### Glossar der Inhaltsstoffe

**Triacylglycerid (MCT-Öl):**
   - Lebensmittelqualität
   - Dichte bei 20°C = 0,95 g/cm³
   - Wassergehalt < 0,05 %
   - Zusammensetzung der Fettsäuren: C6: max 1%; C8: 50-65 %; C10: 34-45 %; C12: max. 2 %; C14: max. 1%
**SiO₂ pyrogen**
   - Lebensmittelqualität
   - BET-Oberfläche: 200 m²/g
   - pH-Wert 5 %ig in Wasser: 4,0
   - Stampfdichte: < 60g/l
**SiO₂ gefällt**
   - Lebensmittelqualität
   - BET-Oberfläche: 160 m²/g
   - pH-Wert 5%ig in Wasser: 7,0
   - SiO₂-Gehalt: > 96%
   - Stampfdichte: 80 g/l
   - Lösliche Salze: < 2,6 %
**Ethylcellulose**
   - Lebensmittelqualität
   - Ethoxylierungsgrad: 49 %
   - Viskosität (5%ig in Toluol/Ethanol 80/20): 190 mpas
   - Restgewicht nach Veraschung: < 0,4%

Die Inhaltsstoffe wurden gemäß Tabelle 1 miteinander vermischt. Die Gewichtsangaben sind in Gewichtsprozent, auf der Basis der Summe des Gewichts aller Bestandteile der Zusammensetzung.

Vor der Vermischung der Inhaltsstoffe wurden die pyrogene Kieselsäure und die gefällte Kieselsäure 120 min lang bei 130 °C vor ausgeheizt, um die Oberflächen zu trocknen. Bis auf Beispiel 2 und 9 wurde SiO₂ mit MCT-Öl bei Raumtemperatur homogenisiert. Die Beispiele 2 und 9 enthalten im Gegensatz zu den anderen Beispielen Ethylcellulose. Zur Herstellung dieser Zusammensetzungen wurde die Ethylcellulose bei 150°C-180°C in MCT-Öl gelöst, danach SiO₂ eingearbeitet, dann auf Raumtemperatur abgekühlt.

**Tabelle 1: Zusammensetzungen**

| | **Erfindungsgemäß** | | | | | | **Vergleich** | | |
|---|---|---|---|---|---|---|---|---|---|
| Gew.-% | **1** | **2** | **3** | **4** | **5** | **6** | **7** | **8** | **9** |
| MCT-Öl | 85,0 | 91,0 | 95,8 | 89,6 | 70,0 | 70,0 | 96,0 | 90,0 | 95,9 |
| SiO₂ pyrogen | 15,0 | 4,0 | 4,0 | - | 30,0 | - | 4,0 | - | - |
| SiO₂ gefällt | - | - | - | 10,0 | - | 30,0 | - | 10,0 | - |
| EthylCellulose | - | 5,0 | - | - | - | - | - | - | 5,0 |
| Wasser dest. | - | - | 0,2 | 0,4 | - | - | - | - | |
| Verhältnis MCT-Öl zu SiO₂ | 1 : 0,18 | 1 : 0,04 | 1 : 0,04 | 1 : 0,11 | 1 : 0,4 | 1 : 0,4 | 1 : 0,04 | 1 : 0,11 | 1 : 0,05 |
| Struktur bei 21°C | Formkörper aus festem Gel | Formkörper aus festem Gel | Gel | Gel | Pulver | Pulver | flüssig | flüssig | Formkörper aus festem Gel |
| Brandverhalten | brennt gut | brennt gut | brennt gut | brennt gut | brennt schwach | brennt schwach | brennt nicht | brennt nicht | brennt nicht |

Die Viskosität der Gele der Beispiele 1 bis 4 und 9 wurde mit einem Brookfield Viskosimeter, Spindel 5 (0,5 Umdrehungen/min) bei 21°C gemessen.

Das Gel aus Beispiel 3 wies eine Viskosität von 350.000 mPa·s auf und das Gel aus Beispiel 4 wies eine Viskosität von 70.000 mPa·s auf.

Bei den Formkörpern aus festem Gel der Beispiele 1, 2 und 9 war die Viskosität nicht messbar, bei mechanischer Einwirkung brachen die Formkörper aus festem Gel und zerfielen in mehrere Teile.

Um das Brandverhalten der Zusammensetzungen aus den Beispielen 1 bis 6 und 9 zu beurteilen, wurden die Zusammensetzungen auf eine Fläche von 10 cm² mit einer Höhe von ca. 0,2 bis 0,5 cm verteilt. Dann wurde die Flamme eines Feuerzeugs mit einer Länge von ca. 4 cm über 4 Sekunden an die jeweilige Probe gehalten. Bei den brennenden Zusammensetzungen haben verschiedene Personen während des Abbrennens jeweils aus einem Abstand von ca. 40-50 cm mit ihrer Atemluft gegen die Flamme geblasen, so als ob man eine Kerze ausbläst. Das Brandverhalten wurde wie folgt kategorisiert:

**Tabelle 2: Brandverhalten der Zusammensetzungen**

| | |
|---|---|
| Brennt nicht | Nach dem dritten Versuch sie nach der oben beschrieben Verfahrensweise anzuzünden, brennt die Probe nicht |
| Brennt schwach | Probe brennt nur langsam ab und die Flamme erlischt schon bei leichtem Luftzug |
| Brennt gut | Probe lässt sich leicht beim ersten Versuch entzünden und brennt nach Wegnahme der Zündquelle selbständig weiter und brennt vollständig ab, wobei die Flamme intensiv ist und nicht durch Ausblasen mit der Atemluft gelöscht werden konnte |

Die brennenden Zusammensetzungen brannten nach Wegnahme der Zündquelle selbstständig weiter. Die Zusammensetzungen, die als "gut brennend" bezeichnet werden, brannten über die gesamte Fläche mit einer gelblichen Flamme von 4 bis 8 cm Höhe gleichmäßig bis zu dem Zeitpunkt, an dem kein MCT-Öl mehr vorhanden war, d.h. sie verbrannten vollständig. Die Flamme konnte nicht durch Ausblasen gelöscht werden, sondern nur indem ein Deckel aus Metall oder Keramik auf die brennende Masse gelegt wurde, so dass die Sauerstoffzufuhr beendet wurde.

Die Zusammensetzungen der Beispiele 2 (erfindungsgemäß) und 9 (Vergleich) unterscheiden sich im wesentlichen dadurch, dass die Zusammensetzung von Beispiel 9 keine Kieselsäure enthält. Die experimentellen Daten zeigen, dass die Zusammensetzung des Vergleichsbeispiels 9 ohne Kieselsäure nicht brennt, während die erfindungsgemäße Zusammensetzung aus Beispiel 2 mit pyrogener Kieselsäure gut brennt. Des Weiteren brennen die flüssigen Zusammensetzungen nicht.

Der Vergleich des erfindungsgemäßen Beispiels 3 mit Vergleichsbeispiel 7 zeigt, dass durch die Kombination einer vergleichsweise geringen Konzentration an Kieselsäure und einem Verdickungsmittel auch brennbare Zusammensetzungen erhältlich sind, die ohne die Zugabe des Verdickungsmittels nicht brennbar wären.

## Patentansprüche

1. Zusammensetzung enthaltend oder bestehend aus
a) von 70 bis 96 Gew.-% Triacylglycerol von gesättigten Fettsäuren, welche eine Alkylkette von 6 bis 12 Kohlenstoffatomen enthalten,
b) von 3,0 bis 30 Gew.-% an Kieselsäure, und
c) optional von 0,1 bis 5,0 Gew.-% wenigstens eines Verdickungsmittels enthaltend wenigstens ein Cellulosederivat und/ oder Wasser,
d) optional von 1,0 bis 35 Gew.-% wenigstens eines Zusatzstoffes, ausgenommen Verdickungsmittel,
jeweils bezogen auf das Gesamtgewicht der Zusammensetzung, wobei das Verhältnis von Triacylglycerol a) zu Kieselsäure b) in einem Bereich von 1 : 0,04 bis 1 : 0,4 liegt,
**dadurch gekennzeichnet, dass**
die Kieselsäure pyrogene Kieselsäure und/oder gefällte Kieselsäure ist, wobei
wenn die Kieselsäure pyrogene Kieselsäure ist oder diese umfasst und in der Zusammensetzung bis zu 4,0 Gew.-% hiervon enthalten sind, die Zusammensetzung 0,1 bis 5,0 Gew.-% des wenigstens einen Verdickungsmittels enthält und
wenn die Kieselsäure gefällte Kieselsäure ist oder diese umfasst und in der Zusammensetzung bis zu 10 Gew.-%, hiervon enthalten sind, die Zusammensetzung 0,1 bis 5,0 Gew.-% des wenigstens einen Verdickungsmittels enthält,
wobei die Zusammensetzung eine dynamische Viskosität nach DIN 53018 von 50.000 bis 400.000 mPa·s, gemessen mit einem Brookfield Viskosimeter, Spindel 5, bei 0,5 Umdrehungen/min bei 21 °C aufweist oder bei Raumtemperatur fest ist und einen Flammpunkt nach EN 22719 von 210°C bis 240°C hat.

2. Zusammensetzung nach Anspruch 1, **dadurch gekennzeichnet, dass** das Verdickungsmittel bei Raumtemperatur ein Feststoff ist.

3. Zusammensetzung nach Anspruch 1 oder 2, **dadurch gekennzeichnet, dass** das Verdickungsmittel weitere Verdickungsmittel umfasst, ausgewählt aus der Gruppe bestehend aus Cellulosefasern, Alginaten, Agar-Agar, Johannisbrotkernmehl, Guarkernmehl, Carrageen, Furcellaran, Xanthan, Pektin, Methylcellulose, Ethylcellulose, Hydroxyethylcellulose, Carboxymethylcellulosen, Hydroxypropylmethylcellulose, Hydroxypropylcellulose, Ethylhydroxyethylcellulose und mikrokristalliner Cellulose sowie Mischungen von diesen, insbesondere ist das Verdickungsmittel Ethylcellulose.

4. Zusammensetzung nach einem der vorstehenden Ansprüche, **dadurch gekennzeichnet, dass** die Zusammensetzung 4,0 bis 30 Gew.-%, bevorzugt 10 bis 20 Gew.- %, pyrogene Kieselsäure enthält.

5. Zusammensetzung nach einem der vorstehenden Ansprüche, **dadurch gekennzeichnet, dass** die Zusammensetzung 10 bis 15 Gew.-%, bevorzugt 11 bis 13 Gew.- %, gefällte Kieselsäure enthält.

6. Zusammensetzung nach einem der vorstehenden Ansprüche, **dadurch gekennzeichnet, dass** die Kieselsäure eine BET-Oberfläche nach DIN ISO 9277 von 150 m²/g bis 250 m²/g, insbesondere von 160 m²/g bis 200 m²/g, aufweist.

7. Zusammensetzung nach einem der vorstehenden Ansprüche, **dadurch gekennzeichnet, dass** der Wassergehalt der Kieselsäure nach DIN EN ISO 787-2 höchstens 3,0 Gew.-% Wasser beträgt, insbesondere von 0,1 Gew.-% bis 0,5 Gew.-%, jeweils bezogen auf die Gesamtmenge Kieselsäure in der Zusammensetzung.

8. Zusammensetzung nach einem der vorstehenden Ansprüche, **dadurch gekennzeichnet, dass** die Zusammensetzung 4,0 bis 10 Gew.-% des wenigstens einen Zusatzstoffes, ausgenommen Verdickungsmittel, enthält.

9. Zusammensetzung nach einem der vorstehenden Ansprüche, **dadurch gekennzeichnet, dass** der Zusatzstoff ausgewählt ist aus der Gruppe bestehend aus
- Nahrungsergänzungsmitteln gemäß den Anlagen 1 und 2 zur Verordnung über Nahrungsergänzungsmittel, vom 24.05.2004, BGBl. I S. 1011, Stand 17.1.2007,
- Lebensmittelzusatzstoffen gemäß Anlage 1 zur Verordnung über die Zulassung von Zusatzstoffen zu Lebensmitteln zu technologischen Zwecken, vom 29.01.1998, BGBl. I S. 230, Stand 05.07.2017, und
- Aromen gemäß der Aromenverordnung vom 2. Mai 2006 (BGBl. I S. 1127), Stand 05.07.2017, sowie Mischungen von diesen.

10. Zusammensetzung nach einem der vorstehenden Ansprüche, **dadurch gekennzeichnet, dass** die Zusammensetzung ausschließlich aus Bestandteilen besteht, die jeweils zugelassen sind
- als Lebensmittel gemäß dem Lebensmittel und Futtermittelgesetzbuch (LFGB) sowie der Verordnung EG 178/2002 (Lebensmittel-Basisverordnung), oder
- als Nahrungsergänzungsmittel gemäß den Anlagen 1 und 2 zur Verordnung über Nahrungsergänzungsmittel, vom 24.05.2004, BGBl. I S. 1011, Stand 17.1.2007, oder
- als Lebensmittelzusatzstoffe gemäß Anlage 1 zur Verordnung über die Zulassung von Zusatzstoffen zu Lebensmitteln zu technologischen Zwecken, vom 29.01.1998, BGBl. I S. 230, Stand 05.07.2017, oder
- als Aromen gemäß der Aromenverordnung vom 2. Mai 2006 (BGBl. I S. 1127), Stand 05.07.2017.

11. Zusammensetzung nach einem der vorstehenden Ansprüche, **dadurch gekennzeichnet, dass** die Zusammensetzung einen Flammpunkt nach EN 22719 von 220 bis 230 °C, hat und vorzugsweise nach Wegnahme der Zündquelle selbstständig weiterbrennt.

12. Zusammensetzung nach einem der vorstehenden Ansprüche, **dadurch gekennzeichnet, dass** die Zusammensetzung eine dynamische Viskosität nach DIN 53018 von 70.000 bis 350.000 mPa·s, gemessen mit einem Brookfield Viskosimeter, Spindel 5, bei 0,5 Umdrehungen/min bei 21 °C aufweist.

13. Zusammensetzung nach einem der vorstehenden Ansprüche, **dadurch gekennzeichnet, dass** die Zusammensetzung enthält oder besteht aus
a) von 85 bis 90 Gew.-% Triacylglycerol von gesättigten Fettsäuren, welche eine Alkylkette von 6 bis 12 Kohlenstoffatomen enthalten,
b) von 4,0 bis 15 Gew.-% an Kieselsäure,
c) optional von 0,1 bis 5,0 Gew.-% des wenigstens einen Verdickungsmittels, und
d) optional 0,1 bis 7,0 Gew.-% des wenigstens einen Zusatzstoffes, ausgenommen Verdickungsmittel,
jeweils bezogen auf das Gesamtgewicht der Zusammensetzung.

14. Formkörper enthaltend eine Zusammensetzung nach einem der Ansprüche 1 bis 13, wobei der Formkörper insbesondere teilweise oder vollständig mit einer Beschichtung überzogen ist und die Beschichtung vorzugsweise eine Alkylcellulose enthält oder hieraus besteht, bevorzugt Ethylcellulose.

15. Verwendung einer Zusammensetzung nach einem der Ansprüche 1 bis 13 oder eines Formkörpers nach Anspruch 14 als Ausrüstungsgegenstand für Outdooraktivitäten, Camping, sportlichen Aktivitäten und/oder Nahrungsmittel für eine sportliche- oder Outdooraktivität.

## Claims

1. Composition comprising or consisting of
a) from 70% to 96% by weight of triacylglycerol of saturated fatty acids that comprise an alkyl chain of 6 to 12 carbon atoms,
b) from 3.0% to 30% by weight of silica, and
c) optionally from 0.1% to 5.0% by weight of at least one thickener comprising at least one cellulose derivative and/or water,
d) optionally from 1.0% to 35% by weight of at least one additive other than thickeners,
in each case based on the total weight of the composition, wherein the ratio of triacylglycerol a) to silica b) is within a range from 1:0.04 to 1:0.4, **characterized in that** the silica is fumed silica and/or precipitated silica, wherein
when the silica is or comprises fumed silica and the composition comprises up to 4.0% by weight thereof, the composition comprises 0.1% to 5.0% by weight of at least one thickener and
when the silica is or comprises precipitated silica and the composition comprises up to 10% by weight thereof, the composition comprises 0.1% to 5.0% by weight of at least one thickener,
wherein the composition has a dynamic viscosity at 21°C in accordance with DIN 53018 of 50 000 to 400 000 mPa·s, measured with a Brookfield viscometer, spindle 5, at 0.5 revolutions per minute, or is solid at room temperature, and a flash point in accordance with EN 22719 of 210°C to 240°C.

2. Composition according to Claim 1, **characterized in that** the thickener is at room temperature a solid.

3. Composition according to Claim 1 or 2, **characterized in that** the thickener includes further thickeners selected from the group consisting of cellulose fibers, alginates, agar-agar, locust bean gum, guar gum, carrageenan, furcellaran, xanthan gum, pectin, methylcellulose, ethylcellulose, hydroxyethylcellulose, carboxymethylcelluloses, hydroxypropyl methylcellulose, hydroxypropylcellulose, ethylhydroxyethylcellulose, and microcrystalline cellulose and also mixtures thereof, in particular the thickener is ethylcellulose.

4. Composition according to any of the preceding claims, **characterized in that** the composition comprises 4.0% to 30% by weight, more preferably 10% to 20% by weight, of fumed silica.

5. Composition according to any of the preceding claims, **characterized in that** the composition comprises 10% to 15% by weight, more preferably 11% to 13% by weight, of precipitated silica.

6. Composition according to any of the preceding claims, **characterized in that** the silica has a BET surface area in accordance with DIN ISO 9277 from 150 m²/g to 250 m²/g, in particular from 160 m²/g to 200 m²/g.

7. Composition according to any of the preceding claims, **characterized in that** the water content of the silica in accordance with DIN EN ISO 787-2 is not more than 3.0% by weight of water, in particular from 0.1% by weight to 0.5% by weight, in each case based on the total weight of silica in the composition.

8. Composition according to any of the preceding claims, **characterized in that** the composition comprises from 4.0% to 10% by weight of at least one additive other than thickeners.

9. Composition according to any of the preceding claims, **characterized in that** the additive is selected from the group consisting of
- food supplements as defined in Annexes 1 and 2 to the Verordnung über Nahrungsergänzungsmittel [German Ordinance on Food Supplements] dated 24.05.2004, BGBl. I [German Federal Law Gazette part I] p. 1011, version of 17.01.2007,
- food additives as defined to Annex 1 to the Verordnung über die Zulassung von Zusatzstoffen zu Lebensmitteln zu technologischen Zwecken [German Ordinance on the Approval of Additives in Food for Technological Purposes] dated 29.01.1998, BGBl. I [German Federal Law Gazette part I] p. 230, version of 05.07.2017, and
- flavorings, as defined in the Aromenverordnung [German Ordinance on Flavorings] dated 2 May 2006 (BGBl. I [German Federal Law Gazette part I] p. 1127), version of 05.07.2017,
and also mixtures of said substances.

10. Composition according to any of the preceding claims, **characterized in that** the composition consists only of constituents that are in each case approved
- as foods, as defined in the Lebensmittel und Futtermittelgesetzbuch [Food and Feed Code] (LFGB) and Regulation EC 178/2002 (General Food Law Regulation), or
- as food supplements, as defined in Annexes 1 and 2 to the Verordnung über Nahrungsergänzungsmittel [German Ordinance on Food Supplements] dated 24.05.2004, BGBl. I [German Federal Law Gazette part I] p. 1011, version of 17.01.2007, or
- as food additives, as defined to Annex 1 to the Verordnung über die Zulassung von Zusatzstoffen zu Lebensmitteln zu technologischen Zwecken [German Ordinance on the Approval of Additives in Food for Technological Purposes] dated 29.01.1998, BGBl. I [German Federal Law Gazette part I] p. 230, version of 05.07.2017, or
- as flavorings, as defined in the Aromenverordnung [German Ordinance on Flavorings] dated 2 May 2006 (BGBl. I [German Federal Law Gazette part I] p. 1127), version of 05.07.2017.

11. Composition according to any of the preceding claims, **characterized in that** the composition has a flash point in accordance with EN 22719 of 220 to 230°C and preferably continues to burn independently after the ignition source has been removed.

12. Composition according to any of the preceding claims, **characterized in that** the composition has a dynamic viscosity at 21°C in accordance with DIN 53018 of 70 000 to 350 000 mPa·s, measured with a Brookfield viscometer, spindle 5, at 0.5 revolutions per minute.

13. Composition according to any of the preceding claims, **characterized in that** the composition comprises or consists of
a) from 85% to 90% by weight of triacylglycerol of saturated fatty acids that comprise an alkyl chain of 6 to 12 carbon atoms,
b) from 4.0% to 15% by weight of silica,
c) optionally from 0.1% to 5.0% by weight of at least one thickener, and
d) optionally 0.1% to 7.0% by weight of at least one additive other than thickeners,
in each case based on the total weight of the composition.

14. Shaped body comprising a composition according to any of Claims 1 to 13, wherein the shaped body is in particular partially or completely covered with a coating and the coating preferably comprises or consists of an alkyl cellulose, preferably ethylcellulose.

15. Use of a composition according to any of Claims 1 to 13 or of a shaped body according to Claim 14 as an item of equipment for outdoor activities, camping, sports activities, and/or a foodstuff for a sports/outdoor activity.

## Revendications

1. Composition contenant, ou en étant constituée :
a) de 70 à 96 % en poids d'un triacylglycérol d'acides gras insaturés, qui contiennent une chaîne alkyle ayant 6 à 12 atomes de carbone,
b) de 3,0 à 30 % en poids de silice, et
c) éventuellement de 0,1 à 5,0 % en poids d'au moins un épaississant contenant au moins un dérivé de la cellulose et/ou de l'eau,
d) éventuellement de 1,0 à 35 % en poids d'au moins un additif, à l'exclusion de l'épaississant,
dans chaque cas par rapport au poids total de la composition, le rapport du triacylglycérol a) à la silice b) étant dans la plage de 1:0,04 à 1:0,4,
**caractérisée en ce que**
la silice est une silice pyrogène et/ou une silice précipitée et est présente dans la composition en une quantité jusqu'à 4,0 % en poids de cette dernière, la composition contenant 0,1 à 5,0 % en poids de l'au moins un épaississant et
quand la silice est une silice précipitée ou la comprend, et est présente dans la composition en une quantité jusqu'à 10 % en poids par rapport à cette dernière, la composition contenant 0,1 à 5,0 % en poids de l'au moins un épaississant,
la composition présentant une viscosité dynamique selon DIN 53018 de 50 000 à 400 000 mPa-s, mesurée à l'aide d'un viscosimètre Brookfield, broche 5 à 0,5 tour/minute à 21 °C, ou est solide à la température ambiante et a un point d'éclair selon EN 22719 de 210 °C à 240 °C.

2. Composition selon la revendication 1, **caractérisée en ce que** l'épaississant est solide à la température ambiante.

3. Composition selon la revendication 1 ou 2, **caractérisée en ce que** l'épaississant comprend d'autres épaississants choisis dans le groupe consistant en les fibres de cellulose, les alginates, l'agar-agar, la gomme de caroube, la gomme de guar, la mousse d'Irlande, le furcellarane, le xanthane, la pectine, la méthylcellulose, l'éthylcellulose, l'hydroxyéthylcellulose, les carboxyméthylcelluloses, l'hydroxypropylméthylcellulose, l'hydroxypropylcellulose, l'éthylhydroxyéthylcellulose et la cellulose microcristalline, ainsi que les mélanges de ceux-ci, en particulier l'épaississant est l'éthylcellulose.

4. Composition selon l'une des revendications précédentes, **caractérisée en ce que** la composition contient 4,0 à 30 % en poids, de préférence 10 à 20 % en poids de silice pyrogène.

5. Composition selon l'une des revendications précédentes, **caractérisée en ce que** la composition contient 10 à 15 % en poids, de préférence 11 à 13 % en poids de silice précipitée.

6. Composition selon l'une des revendications précédentes, **caractérisée en ce que** la silice présente une aire BET selon DIN ISO 9277 de 150 m²/g à 250 m²/g, en particulier de 160 m²/g à 200 m²/g.

7. Composition selon l'une des revendications précédentes, **caractérisée en ce que** la teneur en eau de la silice selon DIN EN ISO 787-2 est d'au plus 3,0 % en poids d'eau, en particulier de 0,1 % en poids à 0,5 % en poids, dans chaque cas par rapport au poids total de la silice dans la composition.

8. Composition selon l'une des revendications précédentes, **caractérisée en ce qu'**elle contient 4,0 à 10 % en poids de l'au moins un additif, à l'exclusion de l'épaississant.

9. Composition selon l'une des revendications précédentes, **caractérisée en ce que** l'additif est choisi dans le groupe consistant en
- les compléments alimentaires selon les annexes 1 et 2 du règlement relatif aux compléments alimentaires, du 24.05.2004, BGBI. !S. 1011, Statut au 17.1.2007,
- les additifs alimentaires selon l'annexe 1 au règlement relatif à l'autorisation des additifs à des denrées alimentaires à des fins technologiques, du 29.01.1998, BGBI. !S. 230, Statut au 05.07.2017, et
- les arômes selon le règlement sur les arômes du 2 mai 2006 (BGBI. IS. 1127), Statut au 05.07.2017, ainsi que les mélanges de ceux-ci.

10. Composition selon l'une des revendications précédentes, **caractérisée en ce que** la composition est constituée exclusivement de constituants qui sont autorisés
- comme denrées alimentaires selon le code des denrées alimentaires et des aliments pour animaux (LFGB), ainsi que selon le règlement EG 178/2002 (règlement de base sur les denrées alimentaires), ou
- comme compléments alimentaires selon les annexes 1 et 2 au règlement sur les compléments alimentaires, du 24.05.2004, BGBI. IS. 1011, Statut au 17.1.2007, ou
- comme additifs alimentaires selon l'annexe 1 au règlement relatif à l'autorisation des additifs aux denrées alimentaires à des fins technologiques, du 29.01.1998, BGBI. IS. 230, Statut au 05.07.2017, ou
- comme arômes selon le règlement sur les arômes du 2 mai 2006 (BGBI. IS. 1127), Statut au 05.07.2017.

11. Composition selon l'une des revendications précédentes, **caractérisée en ce qu'**elle a un point d'éclair selon EN 22719 de 220 à 230 °C et de préférence continue à brûler d'une manière autonome après éloignement de la source d'inflammation.

12. Composition selon l'une des revendications précédentes, **caractérisée en ce qu'**elle présente une viscosité dynamique selon DIN 53018 de 70 000 à 350 000 mPa.s, mesurée à l'aide d'un viscosimètre Brookfield, broche 5 à 0,5 tour/minute à 21 °C.

13. Composition selon l'une des revendications précédentes, **caractérisée en ce qu'**elle contient, ou en est constituée
a) de 85 à 90 % en poids d'un triacylglycérol d'acides gras insaturés, qui contiennent une chaîne alkyle ayant 6 à 12 atomes de carbone,
b) de 4,0 à 15 % en poids de silice,
c) éventuellement de 0,1 à 5,0 % en poids de l'au moins un épaississant, et
d) éventuellement 0,1 à 7,0 % en poids de l'au moins un additif, à l'exclusion de l'épaississant,
dans chaque cas par rapport au poids total de la composition.

14. Objet façonné contenant une composition selon l'une des revendications 1 à 13, l'objet façonné étant en particulier en partie ou complètement revêtu d'un revêtement, et le revêtement contenant de préférence une alkylcellulose ou en étant constituée, de préférence l'éthylcellulose.

15. Utilisation d'une composition selon l'une des revendications 1 à 13 ou d'un objet façonné selon la revendication 14, en tant qu'apprêt pour activités en extérieur, camping, activités sportives et/ou denrées alimentaires pour une activité sportive ou en extérieur.
